# EUROPÄISCHE PATENTSCHRIFT

(11) **EP 2 782 612 B1**
(45) Veröffentlichungstag und Bekanntmachung des Hinweises auf die Patenterteilung: **01.01.2020**
(21) Anmeldenummer: 12788211.6
(22) Anmeldetag: 20.11.2012
(51) Int. Cl.: A61L 29/16, A61L 29/08, A61M 31/00, A61K 31/00, A61K 33/24, A61M 25/00

(54) **VORRICHTUNG ZUM VERHINDERN UND/ODER BEHANDELN VON HARNWEGSINFEKTIONEN**
DEVICE FOR PREVENTING AND/OR TREATING URINARY TRACT INFECTIONS
DISPOSITIF POUR EMPÊCHER ET/OU TRAITER DES INFECTIONS URINAIRES

(30) Priorität: 22.11.2011 DE 102011119160
(43) Veröffentlichungstag der Anmeldung: 01.10.2014
(73) Patentinhaber: Largentec Gmbh, 14129 Berlin (DE)
(72) Erfinder: LANDAU, Uwe, 14169 Berlin (DE)
(74) Vertreter: Remus, Alvaro Johannes
(86) Internationale Anmeldenummer: PCT/EP2012/073123
(87) Internationale Veröffentlichungsnummer: WO 2013/076088

(56) Entgegenhaltungen:
- EP-A2- 0 328 421
- DE-A1-102006 049 108
- US-A1- 2001 008 896
- US-A1- 2007 161 967
- US-A1- 2007 239 107

## Beschreibung

### Hintergrund der Erfindung

Die Erfindung bezieht sich auf eine Vorrichtung zum Verhindern und/oder Behandeln von Harnwegsinfektionen im Bereich des Harnleiters, insbesondere auf eine Vorrichtung mit einem Dorn, der in den Harnleiter einführbar ist. Eine solche Vorrichtung ist z.B. aus US 2007/239107 A1 bekannt.

Harnwegsinfektionen werden meistens ausgelöst durch Bakterien, können aber auch durch Pilze oder Viren verursacht werden. Die Mikroorganismen gelangen von außen in den Harnleiter, bei Frauen über die Scheide, bei Männern über den Penis. Durch Bakterien verursachte Harnwegsinfektionen werden üblicherweise mit Antibiotika behandelt. Fachleute in den USA und in Europa haben festgestellt, dass die Bakterien mehr und mehr resistent gegenüber Antibiotika werden. Die Wirksamkeit von Antibiotika hat in den letzten Jahren durch die Resistenz der Bakterien bis zu 50% und mehr nachgelassen. Harnwegsinfektionen betreffen jedes Jahr Millionen Personen. Auch wenn eine anfängliche Infektion nicht lebensbedrohend ist, können unbehandelte oder nicht sachgemäß behandelte Harnwegsinfektionen zu Blasenschädigungen und im Weiteren zu Niereninfektionen führen. Hierbei treten oftmals Komplikationen auf, so dass die befallene Person im Krankenhaus behandelt werden muss. Rezidivierende, d.h. wiederkehrende Harnwegsinfektionen treten bei etwa 2 bis 5% der Frauen auf, wobei bei Männern das Auftreten etwa um die Hälfte niedriger ist. Etwa 40% von inkontinenten, insbesondere älteren Personen leiden an Harnwegsinfektionen, wobei diese Rate bis zu 60% erhöht ist, wenn diese Personen bereits Katheter zur Linderung etwaiger Blaseninkontinenz verwenden. Die Behandlung der Patienten mit Harnwegsinfektionen ist zeit- und kostenaufwendig, da der Patient ständig überwacht werden muss, viele Hausbesuche eines Arztes nötig und Arzneimittel, insbesondere teure Antibiotika, erforderlich sind.

### Beschreibung der Erfindung

Der Erfindung liegt die Aufgabe zugrunde, eine Vorrichtung zum Verhindern und/oder Behandeln von Harnwegsinfektionen im Bereich des Harnleiters anzugeben, die einfach aufgebaut und hochwirksam ist und darüber hinaus von den Patienten selbständig ohne ärztliche Hilfe angewendet werden kann.

Diese Aufgabe wird erfindungsgemäß durch eine Vorrichtung der eingangs genannten Art gelöst, bei der der Dorn, der in den Harnleiter einführbar ist, eine antimikrobielle Beschichtung umfasst, die Silber und Ruthenium sowie mindestens ein Vitamin oder mindestens ein Derivat eines Vitamins umfasst, wobei das Vitamin mindestens eine Verbindung aus der Gruppe der wasserlöslichen Vitamine oder ein Salz oder saures Derivat dieser Verbindung ist.

Mit der Erfindung wird ein handliches kleines Instrument zur Verfügung gestellt, das auch von einem Patienten selbst ohne ärztliche Aufsicht angewendet werden kann, um eine Harnwegsinfektion zu verhindern oder zu behandeln. Zu diesem Zweck kann mit dem Dorn ein Handgriff zum Manipulieren des Dorns und damit der Vorrichtung verbunden sein.

Auch wenn die erfindungsgemäße Vorrichtung vom Patienten selbstständig angewendet werden kann, sollte eine überwachende Betreuung durch einen Arzt oder eine Ärztin selbstverständlich gewährleistet sein.

Bei der Anwendung der Vorrichtung wird die Einführtiefe des Dorns für den Patienten durch ein Grenzelement angegeben, z. B. eine Markierung auf dem Dorn.

Bevorzugt wird die Einführtiefe des Dorns jedoch durch ein mit dem Dorn verbundenes Anschlagelement bestimmt, z.B. eine Platte, die den Dorn in Richtung auf den Handgriff abschließt und sich bei einem Mann an den Peniskopf und bei einer Frau an die äußeren Schamlippen anlegt.

Das Grenz- oder Anschlagelement ist gleichzeitig eine Sterilbarriere, so dass Keime des Patienten während der Manipulation der Vorrichtung nicht von dem Handgriff zu dem Dorn wandern können.

Der Dorn kann beispielsweise mit einem Wirkstoff beschichtet sein, der bei einem Kontakt mit einem Bakterium dessen Zellmembran zerstört und das Bakterium somit deaktiviert. Ein solches Material ist etwa in der EP 1881763 B1 oder der DE 10 2005 020 327 A1 beschrieben.

Die Vorrichtung gemäß der Erfindung kann leicht von einer Patientin oder einem Patienten selbst, ohne ärztliche Hilfe, in den Harnleiter eingeführt werden.

Die Vorrichtung gemäß der Erfindung ist vorzugsweise eine lineare Anordnung aus dem Dorn, einem Grenz- bzw. Anschlagelement und dem Handgriff.

Der Dorn und der Handgriff sind bevorzugt als zylindrische Stäbe ausgebildet.

Insbesondere für männliche Patienten sollte der Dorn flexibel ausgebildet sein, d.h. entweder aus einem relativ elastischen Material, z.B. einem Kunststoff, gefertigt sein oder bei einem relativ starren Material zur Erhöhung seiner Flexibilität das Material lokal schwächende Stellen aufweisen. Möglich ist hierzu zumindest eine oder auch mehrere in Umfangsrichtung verlaufende Rillen vorzusehen.

Es ist auch möglich, mit der Vorrichtung gemäß der Erfindung etwaige Mikroorganismen in dem Harnleiter durch entsprechende Arzneimittel zu deaktivieren. Hierzu wird bevorzugt der Handgriff der Vorrichtung als Behälter ausgebildet, der mit einem Längskanal in dem Dorn kommuniziert. Von diesem Längskanal gehen nach außen führende Abzweigungen aus. Der Behälter in dem Handgriff ist mit einem antimikrobiellen Mittel gefüllt, wobei dieses Mittel aus dem Behälter über die Abzweigungen in den Harnleiter einbringbar ist. Dies geschieht z.B. dadurch, dass der Handgriff aus einem elastischen Material besteht, so dass das Arzneimittel aus dem Behälter in den Harnleiter hinausgedrückt werden kann. Andere Lösungen sind natürlich möglich.

Die Vorrichtung gemäß der Erfindung wird bevorzugt als einstückiges Spritzgussteil aus Kunststoff ausgebildet. Damit wird die Herstellung der Vorrichtung vereinfacht; ebenso sind die Herstellungskosten niedrig.

Erfindungsgemäß ist vorgesehen, dass die antimikrobielle Beschichtung Silber und Ruthenium sowie mindestens ein Vitamin oder mindestens ein Derivat eines Vitamins umfasst, wobei das Vitamin mindestens eine Verbindung aus der Gruppe der wasserlöslichen Vitamine oder ein Salz oder saures Derivat dieser Verbindung ist. Eine solche Beschichtung wurde in DE 10 2006 049 108 A1, als vorteilhaft zur Herstellung einer bioaktiver Vorrichtung für Entkeimung von Wasser oder wässrigen Lösungen beschrieben. Eine mögliche therapeutische Anwendung dieser Beschichtung wurde hier jedoch nicht erkannt.

Bei der erfindungsgemäßen Beschichtung wird, anders als beim bekannten oligodynamischen Effekt des Silbers, die Keimreduktion aufgrund eines völlig anderen Wirkmechanismus erheblich verbessert bzw. durch die Kombination von Silber mit Ruthenium und einem Vitamin, vorzugsweise Ascorbinsäure oder deren Derivaten, erheblich verstärkt. Diese bioaktiven Metalloberflächen bzw. Beschichtungen führen zu einer schnelleren und effizienteren Abtötung von Mikroorganismen. Gleichzeitig verhindern solche Beschichtungen die Besiedelung des Harnleiters mit Mikroorganismen und die Anheftung oder stabile Ablagerung von Biomolekülen, wie DNA, RNA oder Proteinen.

Die antimikrobielle Beschichtung kann zusätzlich vorteilhafterweise mindestens eine oberflächenaktive Substanz umfassen. Die oberflächenaktive Substanz ist dann vorzugsweise mindestens eine Verbindung aus der Gruppe der anionischen, nichtionischen, amphoteren oder kationischen Tenside oder eine geeignete Mischung dieser Verbindungen.

Die antimikrobielle Beschichtung kann ferner Silber-Ruthenium-Bimetall-Partikel umfassen, die mit dem Vitamin bzw. Vitaminderivat nachbehandelt wurden.

Die Beschichtung der erfindungsgemäßen Vorrichtung kann beispielsweise durch das Aufbringen einer Ruthenium-Beschichtung auf eine silberhaltige Oberfläche der Vorrichtung, oder Aufbringen einer Silber-Beschichtung auf die Vorrichtung und anschließend Aufbringen einer Ruthenium-Beschichtung auf die Silber-Beschichtung, oder Aufbringen von Ruthenium-Silber-Partikeln auf die Vorrichtung erfolgen. Die derart beschichtete Oberfläche der Vorrichtung wird dann durch das Aufbringen von mindestens einem Vitamin oder mindestens einem Derivat eines Vitamins und optional mindestens einer oberflächenaktiven Substanz aktiviert. Die Beschichtungen, d.h. die Ruthenium-Beschichtung und eine ggf. erforderliche Silberunterschicht (i.d.R. mit einer Dicke von 2-10 µm), werden vorzugsweise galvanisch aufgebracht oder abgeschieden. Andere Beschichtungsverfahren, wie PVD-, CVD-, Sputter-, Sol-, Gel- und Reduktionsverfahren, sind ebenfalls hierfür geeignete Plattierverfahren.

Die Silber-/Ruthenium-Oberfläche der erfindungsgemäßen Vorrichtung wird mit einem wasserlöslichen Vitamin und vorzugsweise einer oberflächenaktiven Substanz behandelt bzw. umfasst den oder die zusätzlichen Stoffe, so dass die Oberfläche durch diese Stoffe aktiviert wird und eine unerwartet starke antimikrobielle Wirkung der Oberfläche eintritt. Überraschender Weise hat sich herausgestellt, dass nur ein nicht abgebautes wasserlösliches Vitamin die unerwartete antimikrobielle Wirkung im Zusammenspiel mit Silber und Ruthenium bewirkt. Aus vielen Messungen hat sich ergeben, dass sich durch die Nachbehandlung einer mit Ruthenium beschichteten Silberoberfläche mit Ascorbinsäure und beispielsweise einer oberflächenaktiven Substanz die Oberfläche signifikant verändert und die antimikrobielle Wirkung nach einem ganz anderen Mechanismus als bei der klassischen Silbertechnologie (klassische, oligodynamische Silbertechnologie = Abgabe von Silberionen) abläuft. Es gibt Hinweise darauf, dass die Wirkung der antimikrobiellen Beschichtung der erfindungsgemäßen Vorrichtung von einem mikroelektrischen Feld ausgeht und das Benetzungsverhalten der Oberfläche beispielsweise deutlich verändert ist, wenn man eine reine Silber-/Ruthenium-Oberfläche mit der erfindungsgemäßen Oberfläche vergleicht. Bei EM-Atom-Kraft-Mikroskop- Messungen wurden ebenfalls entscheidende Unterschiede festgestellt, die einen deutlichen Hinweise darauf geben, dass sich durch die Kombination von Silber/Ruthenium mit einem wasserlöslichen Vitamin und optional einer oberflächenaktiven Substanz ein mikroelektrisches Feld aufbaut, das bei reinen Silber-/Ruthenium -Oberflächen nicht beobachtet wird. Gleichzeitig bildet sich durch die erfindungsgemäße Beschichtung eine chemisch sehr stabile Oberfläche aus, die z.B. gegen Sulfide und Chloride reaktionsfest ist. Die hohe Stabilität gepaart mit einer langanhaltenden, starken antimikrobiellen Wirksamkeit ist durch mehrjährige antimikrobielle Einsätze der erfindungsgemäßen Beschichtung in diversen wässrigen Lösungen (z.B. Kühlschmiermittellösungen, Prozesswässern mit Korrosionsschutzmitteln und Emulgatoren der unterschiedlichsten Art oder Urin) bestätigt worden.

Bei der Oberfläche der erfindungsgemäßen Vorrichtung, die vorzugsweise eine silberhaltige Oberfläche oder Silberoberfläche umfasst, kann es sich um die Oberfläche eines entsprechenden Kompakt- oder Vollmaterials handeln. Die erfindungsgemäße Vorrichtung kann jedoch auch ein beliebiges anderes Material (wie z.B. Kunststoff) mit einer aufgebrachten, dünnen, silberhaltigen Beschichtung umfassen, die zusammen mit der aufgebrachten äußeren Ruthenium-Beschichtung ein wirksames Ruthenium-Silber-Sandwich-System mit einer Silber- oder Silberlegierungs-Unterschicht oder -Unterlage und einer feuchtigkeits- oder nässedurchlässigen Ruthenium-Außenschicht, -Oberschicht oder -Deckschicht bildet.

Die Erfindung wird im Folgenden anhand der Abbildungen beispielhaft näher erläutert.

### Kurze Beschreibung der Abbildungen

Es zeigt:
**Figur 1** eine perspektivische Ansicht einer Vorrichtung gemäß der Erfindung zum Einführen in den Harnleiter einer Frau;
**Figur 2** eine weitere perspektivische Darstellung einer Vorrichtung gemäß der Erfindung zum Einführen in den Harnleiter eines Mannes;
**Figur 3** eine Teildarstellung eines Dornes für die gezeigte Vorrichtung gemäß der Erfindung zur Erhöhung seiner Flexibilität;
**Figur 4** eine weitere Darstellung eines Dornes zur Erhöhung seiner Flexibilität;
**Figur 5** eine weitere Ausführungsform einer Vorrichtung gemäß der Erfindung zur Freigabe eines Arzneimittels in den Harnleiter eines Patienten;
**Figur 6** fotographische Abbildungen einer erfindungsgemäßen Vorrichtung auf einem mit Bakterien geimpften Nährmedium (Suspensionskultur mit Bakterien von *E. coli* (RRI): 10⁷ Zellen/ml mit 200 µl ausplattiert auf einer LB-Agar-Platte, Inkubation der Platte bei 37°C für 18 Stunden) als Ergebnis einer Langzeitstudie über fast 2 Jahre bei einem Probanden:
   a) Erfindungsgemäße Vorrichtung aus Silber (105 mm, 4mm Ø) ohne Agar-Platte;
   b) Unbenutzte erfindungsgemäße Vorrichtung auf Agar-Platte;
   c) Erfindungsgemäße Vorrichtung nach 21 Monaten Anwendung auf Agar-Platte;
   d) Benutzte erfindungsgemäße Vorrichtung nach 4 Stunden Regeneration auf Agar-Platte;

### Beschreibung beispielhafter und bevorzugter Ausführungsformen der Erfindung

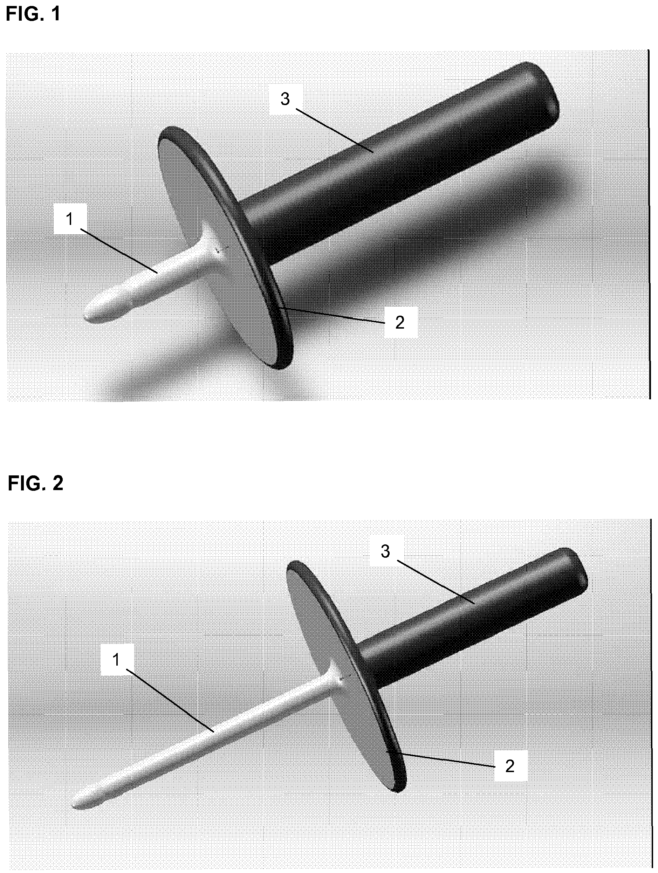

Gemäß den **Figuren 1 und 2** besteht die Vorrichtung aus einem stabförmigen zylindrischen Dorn 1, einem anschließenden scheibenförmigen Grenz- bzw. Anschlagelement 2 und einem zylindrischen Handgriff 3. Diese drei Elemente sind linear aufeinanderfolgend angeordnet.

Die gesamte Vorrichtung ist ein einstückiges Kunststoff-Spritzteil vorzugsweise aus medizinischem Polyurethan, Polyvinylchlorid, Polyethylen, Polypropylen, Polyamid, Silikon oder einem anderen Thermoplasten mit geeigneten Eigenschaften.

Da der Harnleiter bei Frauen relativ kurz ist, ist gemäß Figur 1 der Dorn nur wenige Zentimeter lang, in der Regel zwischen 2 und 3 Zentimetern.

Nachdem der Harnleiter bei einem Mann wegen des Penis deutlich länger ist, ist der Dorn entsprechend Figur 2 länger ausgebildet und hat eine Länge von etwa 5 Zentimetern.

Der Dorn 1 ist bei beiden Ausführungsformen mit einem oberflächenaktiven Wirkstoff beschichtet, der die Zellmembranen etwaiger Bakterien in dem Harnleiter zerstört und in der Zellmembran Löcher bildet, sodass ggf. andere Mittel in die Zelle eindringen können, um diese Zelle zu zerstören und das Bakterium zu deaktivieren.

Die Beschichtung des Dornes 1 ist z.B. zunächst eine Silberbeschichtung mit einer mikroporösen Struktur. Anschließend wird die Silberschicht mit einem zusätzlichen Edelmetall, hier Ruthenium, über einen elektrochemischen Prozess dotiert und die Oberfläche durch einen Beizprozess nachbehandelt, d. h. beispielsweise durch Benetzung mit einer Ascorbinsäure-Lösung aktiviert.

Zusätzlich können noch Metall-Ionen mit oligodynamischem Effekt, Vitaminkomponenten, oberflächenaktive Verbindungen und Gleitunterstützer zugesetzt werden.

Eine solche Beschichtung hat den Vorteil, dass allein durch den Kontakt mit der Zellmembran des Bakteriums die Zellmembran zerstört und das Bakterium deaktiviert wird.

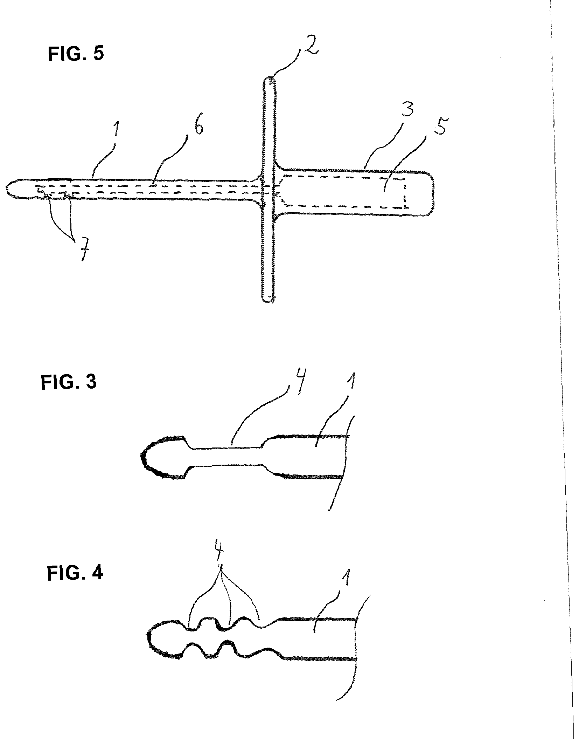

Insbesondere bei einer Vorrichtung für männliche Patienten sollte der Dorn 1, der ja in die Harnröhre in dem Penis eingeführt wird, relativ flexibel sein. Die Flexibilität kann bei einem relativ steifen Material des Dorns u.a., wie in den **Figuren 3 und 4** dargestellt, dadurch erreicht werden, dass das Material des Dorns an einigen Stellen geschwächt wird.

Hierzu kann der Dorn 1 z.B. eine oder mehrere Rillen 4 in Umfangsrichtung aufweisen. Durch die Materialschwächung des Dornes im Bereich der Rillen wird dessen Flexibilität erhöht. Andere Lösungen sind natürlich möglich.

In Figur 3 ist eine relativ breite Rille 4 gezeigt, in der Figur 4 sind mehrere, in diesem Falle drei kurz aufeinanderfolgende Rillen 4 gezeigt.

In **Figur 5** ist eine Vorrichtung gemäß der Erfindung gezeigt, mit der ein Arzneimittel zum Deaktivieren von Bakterien in dem Harnleiter in diesen eingebracht werden kann. Hierzu ist der Handgriff 3 aus einem elastischen Material und ist, wie gestrichelt dargestellt, hohl, sodass ein Behälter 5 gebildet wird. Dieser Behälter 5 kommuniziert mit einem Längskanal 6 in dem Dorn 1. Von diesem Längskanal 6 gehen mehrere Abzweigungen 7 nach außen. Der Behälter 5 ist mit einem Mittel zum Deaktivieren der Bakterien gefüllt. Durch Zusammendrücken des Handgriffes 3 wird dieses Mittel in den Kanal 6 und über die Abzweigungen 7 in den Harnleiter eingebracht, sodass die dort vorhandenen Bakterien deaktiviert werden.

Die geschilderten Ausführungsformen sind beispielhaft. So könnte z.B. der Handgriff 3 und ebenso das Anschlagelement 2 ballonförmig ausgebildet werden, oder der Handgriff mit dem Anschlagelement 2 so verbunden sein, dass die drei Elemente keine lineare Anordnung bilden.

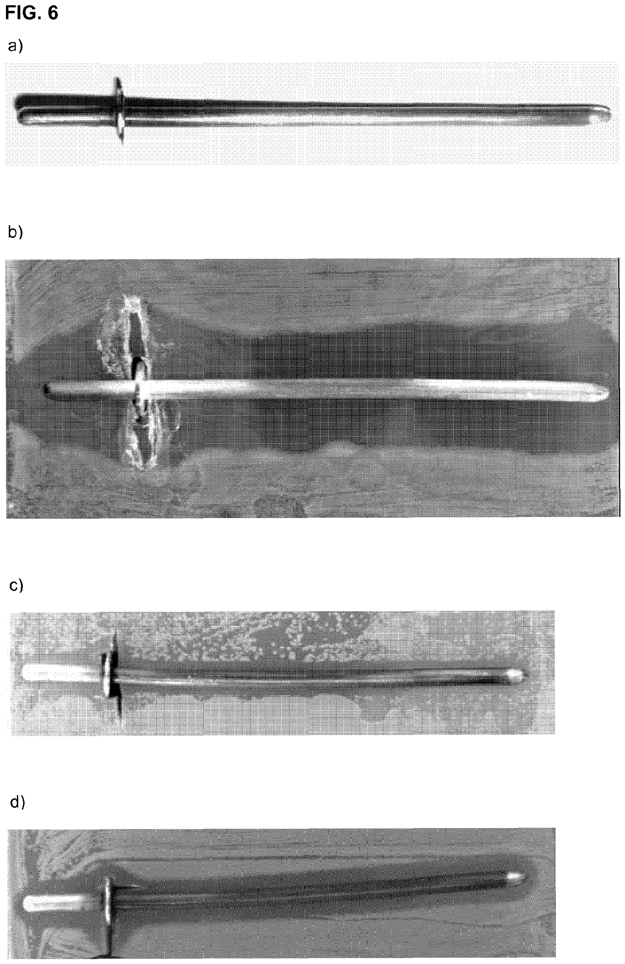

**Figur 6** zeigt fotographische Abbildungen einer erfindungsgemäßen Vorrichtung vor und nach längerer Anwendung sowie nach erfolgter Regeneration. Die erfindungsgemäße Vorrichtung (a) in diesem Test war eine stiftförmige Vorrichtung aus Silber, die aus einem Dorn zum Einführen in den Harnleiter eines Mannes, einem plattenförmigen Anschlagelement zum Begrenzen der Einführtiefe des Dorns und einem Handgriff zum Manipulieren der Vorrichtung bestand. Der aus Silber bestehende Dorn wurde mit Ruthenium beschichtet und diese beschichtete Oberfläche wurde mit einer Lösung, die Ascorbinsäure und eine oberflächenaktive Substanz enthielt, aktiviert. Die Aktivierung wird mit Lösungen durchgeführt, die mit der Silber-/Ruthenium-Beschichtung kompatibel sind. Im einfachsten Fall besteht die Aktivierungslösung aus einer 0,05 M Ascorbinsäurelösung ohne oder mit einem Tensidzusatz. Als Aktivierungslösung kann beispielsweise auch eine Lösung mit 1 mM - 100 mM (vorzugsweise 50 mM) Ascorbinsäure, 1 mM bis 50 mM (vorzugsweise 10 mM) FeCl₃ und 0.1 % bis 5 % (vorzugsweise 0,5 %) SDS verwendet werden. Zur Aktivierung wird der mit Silber und Ruthenium beschichtete Dorn beispielsweise für 2-4 h in die Aktivierungslösung eingetaucht und dann intensiv mit entionisiertem Wasser gewaschen und anschließend getrocknet. Die unbenutzte Vorrichtung zeigte eine starke antimikrobielle Wirkung mit ausgeprägtem Hemmhof auf der beimpften Agar-Platte (b). Dies belegt die antimikrobielle bzw. antibakterielle Wirkung der Beschichtung der erfindungsgemäßen Vorrichtung. Die Behandlung eines männlichen Probanden mit der erfindungsgemäßen Vorrichtung verlief über 21 Monate erfolgreich und ohne Komplikationen. In dieser Zeit der Behandlung konnten Harnwegsinfektionen effektiv verhindert werden. Die Anwendung der erfindungsgemäßen Vorrichtung war problemlos und konnte durch den Probanden selbstständig vorgenommen werden. Nach etwas weniger als zwei Jahren Behandlung des Probanden mit dieser Vorrichtung zeigte diese eine starke dunkle Verfärbung, insbesondere an den Kontaktflächen mit der Harnröhre. Die benutzte Vorrichtung zeigte eine deutliche Reduktion der antimikrobiellen Effizienz, die mit der deutlichen schwärzlichen Verfärbung der Oberfläche korrelierte (c). Eine Nachbehandlung der Vorrichtung für 4 Stunden mit der aktivierenden Lösung führte zu einer leichten Steigerung der antimikrobiellen Aktivität, konnte aber den originalen Zustand der optimalen antimikrobiellen Aktivität nicht wieder herstellen und veränderte auch nicht die dunkle Verfärbung der Oberfläche (d). Dies zeigt, dass zwar grundsätzlich eine Regeneration der antimikrobiellen Wirkung der Beschichtung möglich ist, diese aber nicht ausreichend sein könnte, um eine weitere Behandlung eines Patienten mit gleichbleibender Effizienz zu gewährleisten. Erfindungsgemäß bevorzugt ist daher die Bereitstellung von Einweg-Vorrichtungen, die für eine begrenzte Anzahl von Behandlungen bzw. eine begrenzte Behandlungsdauer vorgesehen sind und dann verworfen und gegen eine neue Vorrichtung ausgetauscht werden.

## Patentansprüche

1. Vorrichtung zum Verhindern und/oder Behandeln von Harnwegsinfektionen im Bereich des Harnleiters, mit einem Dorn (1), der in den Harnleiter einführbar ist, wobei der Dorn (1) eine antimikrobielle Beschichtung umfasst, **dadurch gekennzeichnet, dass** die antimikrobielle Beschichtung Silber und Ruthenium sowie mindestens ein Vitamin oder mindestens ein Derivat eines Vitamins umfasst, wobei das Vitamin mindestens eine Verbindung aus der Gruppe der wasserlöslichen Vitamine oder ein Salz oder saures Derivat dieser Verbindung ist.

2. Vorrichtung nach Anspruch 1, **gekennzeichnet durch** einen mit dem Dorn (1) verbundenen Handgriff (3) zum Manipulieren der Vorrichtung.

3. Vorrichtung nach Anspruch 2, **dadurch gekennzeichnet, dass** zum Angeben der Einführtiefe des Dorns (1) in den Harnleiter der Dorn mit einem Grenzelement (2) versehen ist und dass zum Begrenzen der Einführtiefe des Dorns (1) in den Harnleiter das Grenzelement (2) ein räumliches, den Dorn (1) in Richtung auf den Handgriff (3) abschließendes Anschlagelement ist.

4. Vorrichtung nach Anspruch 3, **dadurch gekennzeichnet, dass** das Anschlagelement eine Platte ist.

5. Vorrichtung nach einem der vorhergehenden Ansprüche, **dadurch gekennzeichnet, dass** die Vorrichtung eine lineare Anordnung aus dem Dorn (1), einem Grenzelement (2) zum Begrenzen der Einführtiefe des Dorns (1) in den Harnleiter und dem Handgriff (3) ist.

6. Vorrichtung nach einem der vorhergehenden Ansprüche, **dadurch gekennzeichnet, dass** der Handgriff (3) und der Dorn (1) jeweils zylindrische Stäbe sind.

7. Vorrichtung nach einem der vorhergehenden Ansprüche, **dadurch gekennzeichnet, dass** der Dorn (1) flexibel ist.

8. Vorrichtung nach Anspruch 7, **dadurch gekennzeichnet, dass** der Dorn (1) zur Erhöhung seiner Flexibilität zumindest eine in Umfangsrichtung verlaufende Rille (4) aufweist.

9. Vorrichtung nach einem der vorhergehenden Ansprüche, **dadurch gekennzeichnet, dass** der Handgriff (3) als Behälter (5) ausgebildet ist, der mit einem Längskanal (6) in dem Dorn (1) kommuniziert, und dass von diesem Längskanal (6) nach außen führende Abzweigungen (7) ausgehen, wobei der Behälter (5) mit einem antimikrobiellen Mittel gefüllt ist, das aus dem Behälter (5) über die Abzweigungen (7) in den Harnleiter einbringbar ist.

10. Vorrichtung nach einem der vorhergehenden Ansprüche, **dadurch gekennzeichnet, dass** die Vorrichtung ein einstückiges Spritzgussteil aus Kunststoff ist.

11. Vorrichtung nach einem der vorhergehenden Ansprüche, **dadurch gekennzeichnet, dass** das Vitamin Ascorbinsäure oder ein Derivat der Ascorbinsäure ist.

12. Vorrichtung nach einem der vorhergehenden Ansprüche, **dadurch gekennzeichnet, dass** die antimikrobielle Beschichtung zusätzlich mindestens eine oberflächenaktive Substanz umfasst.

13. Vorrichtung nach Anspruch 12, **dadurch gekennzeichnet, dass** die oberflächenaktive Substanz mindestens eine Verbindung aus der Gruppe der anionischen, nichtionischen, amphoteren oder kationischen Tenside oder eine geeignete Mischung dieser Verbindungen ist.

14. Vorrichtung nach einem der vorhergehenden Ansprüche, **dadurch gekennzeichnet, dass** die die antimikrobielle Beschichtung Silber-Ruthenium-Bimetall-Partikel umfasst.

## Claims

1. Device for avoiding and/or treating of urinary tract infections in the ureter region, having a mandrel (1) which can be inserted into the ureter, wherein the mandrel (1) comprises an antimicrobial coating, **characterized in that** the antimicrobial coating comprises silver and ruthenium as well as at least one vitamin or at least one derivative of a vitamin, wherein the vitamin is at least one compound from the group of water-soluble vitamins or a salt or acidic derivative of this compound.

2. Device according to claim 1, **characterized by** a hand grip (3) joined to the mandrel (1) for manipulating the device.

3. Device according to claim 2, **characterized in that** the mandrel is provided with a boundary element (2) for indicating the insertion depth of the mandrel (1) in the ureter and that the boundary element (2) is a three-dimensional stopper element terminating the mandrel (1) towards the hand grip (3) for limiting the insertion depth of the mandrel (1) in the ureter.

4. Device according to claim 3, **characterized in that** the stopper element is a plate.

5. Device according to any one of the preceding claims, **characterized in that** the device is a linear arrangement of the mandrel (1), a boundary element (2) for limiting the insertion depth of the mandrel (1) in the ureter, and the hand grip (3).

6. Device according to any one of the preceding claims, **characterized in that** the hand grip (3) and the mandrel (1) each are cylindric rods.

7. Device according to any one of the preceding claims, **characterized in that** the mandrel (1) is flexible.

8. Device according to claim 7, **characterized in that**, for enhancing its flexibility, the mandrel (1) features at least one notch (4) extending in circumferential direction.

9. Device according to any one of the preceding claims, **characterized in that** the hand grip (3) is designed as container (5) which is linked to an axial canal (6) within the mandrel (1) and that branches (7) leading to the outside extend from this axial canal (6), wherein the container (5) is filled with an antimicrobial agent which can be introduced via the branches (7) from the container (5) into the ureter.

10. Device according to any one of the preceding claims, **characterized in that** the device is a one-piece injection moulded part made of plastic material.

11. Device according to any one of the preceding claims, **characterized in that** the vitamin is ascorbic acid or a derivative of ascorbic acid.

12. Device according to any one of the preceding claims, **characterized in that** the antimicrobial coating additionally comprises at least one surface-active substance.

13. Device according to claim 12, **characterized in that** the surface-active substance is at least one compound from the group of anionic, non-ionic, amphoteric, or cationic tensides, or a suitable mixture of these compounds.

14. Device according to any one of the preceding claims, **characterized in that** the antimicrobial coating comprises silver/ruthenium bimetallic particles.

## Revendications

1. Dispositif, destiné à éviter et/ou à traiter des infections urinaires dans la région de l'uretère, pourvu d'un mandrin (1), susceptible d'être introduit dans l'uretère, le mandrin (1) comprenant un revêtement antimicrobien, **caractérisé en ce que** le revêtement antimicrobien comprend de l'argent et du ruthénium, ainsi qu'au moins une vitamine ou au moins un dérivé d'une vitamine, la vitamine étant au moins un composé du groupe des vitamines hydrosolubles ou un sel ou un dérivé acide dudit composé.

2. Dispositif selon la revendication 1, **caractérisé par** un manche (3) relié avec le mandrin (1) pour manipuler le dispositif.

3. Dispositif selon la revendication 2, **caractérisé en ce que** pour indiquer la profondeur d'introduction du mandrin (1) dans l'uretère, le mandrin est muni d'un élément limiteur (2) et **en ce que** pour limiter la profondeur d'introduction du mandrin (1) dans l'uretère, l'élément limiteur (2) est un élément de butée physique, terminant le mandrin (1) dans la direction vers le manche (3).

4. Dispositif selon la revendication 3, **caractérisé en ce que** l'élément de butée est une plaque.

5. Dispositif selon l'une quelconque des revendications précédentes, **caractérisé en ce que** le dispositif est une configuration linéaire composée du mandrin (1), d'un élément limiteur (2), destiné à limiter la profondeur d'introduction du mandrin (1) dans l'uretère et du manche (3).

6. Dispositif selon l'une quelconque des revendications précédentes, **caractérisé en ce que** le manche (3) et le mandrin (1) sont chacun des tiges cylindriques

7. Dispositif selon l'une quelconque des revendications précédentes, **caractérisé en ce que** le mandrin (1) est souple.

8. Dispositif selon la revendication 7, **caractérisé en ce que** pour augmenter sa souplesse, le mandrin (1) comporte au moins une cannelure (4) s'écoulant dans la direction circonférentielle.

9. Dispositif selon l'une quelconque des revendications précédentes, **caractérisé en ce que** le manche (3) est conçu sous la forme d'un réservoir (5) qui communique avec un conduit longitudinal (6) dans le mandrin (1) et **en ce que** de ce conduit longitudinal (6) partent des ramifications (7) menant vers l'extérieur, le réservoir (5) étant rempli d'un produit antimicrobien qui est susceptible d'être introduit dans l'uretère à partir du réservoir (5), par l'intermédiaire des ramifications (7).

10. Dispositif selon l'une quelconque des revendications précédentes, **caractérisé en ce que** le dispositif est une pièce en matière plastique, moulée par injection en monobloc.

11. Dispositif selon l'une quelconque des revendications précédentes, **caractérisé en ce que** la vitamine est de l'acide ascorbique ou un dérivé de l'acide ascorbique.

12. Dispositif selon l'une quelconque des revendications précédentes, **caractérisé en ce que** le revêtement antimicrobien comprend additionnellement une substance active en surface.

13. Dispositif selon la revendication 12, **caractérisé en ce que** la substance active en surface est au moins un composé du groupe des agents tensioactifs anioniques, non ioniques, amphotères ou cationique ou un mélange adapté desdits composés.

14. Dispositif selon l'une quelconque des revendications précédentes, **caractérisé en ce que** le revêtement antimicrobien comprend des particules bimétalliques d'argent/de ruthénium.
